Europäisches Patentamt

European Patent Office

Office européen des brevets

(11) Veröffentlichungsnummer: **0 343 432**
**A2**

(12) **EUROPÄISCHE PATENTANMELDUNG**

(21) Anmeldenummer: 89108415.4

(51) Int. Cl.⁴: **A61B 8/08**

(22) Anmeldetag: 10.05.89

(30) Priorität: 25.05.88 DE 3817726

(43) Veröffentlichungstag der Anmeldung:
29.11.89 Patentblatt 89/48

(84) Benannte Vertragsstaaten:
DE FR GB NL

(71) Anmelder: Siemens Aktiengesellschaft
Wittelsbacherplatz 2
D-8000 München 2(DE)

(72) Erfinder: Granz, Bernd, Dr.
Leonhardstrasse 6
D-8501 Oberasbach(DE)

(54) Vorrichtung zur räumlichen Ultraschall-Ortung von Konkrementen.

(57) Die Erfindung bezieht sich auf eine Vorrichtung zur räumlichen Ultraschall-Ortung von im Körper (2) eines Lebewesens befindlichen Konkrementen (4), die zur Zertrümmerung in einem Lithotriptor im Fokus (F) einer Ultraschall-Stoßwelle (8) angeordnet werden. Erfindungsgemäß ist zur Ultraschall-Ortung eine in Reflexion betriebene Ultraschall-Kamera vorgesehen, deren Schärfenbereich (B) auf eine Gegenstandsebene (32) eingestellt ist, die den Fokus (F) der Ultraschall-Stoßwelle (8) enthält. Dadurch können dem Benutzer Informationen aus Schnittebenen des Körpers (2) zur Verfügung gestellt werden, die eine sichere Positionierung des Konkrementes (4) im Fokus (F) der Ultraschall-Stoßwelle (8) erleichtern.

FIG 1

## Vorrichtung zur räumlichen Ultraschall-Ortung von Konkrementen

Die Erfindung bezieht sich auf eine Vorrichtung zur räumlichen Ultraschall-Ortung von Konkrementen gemäß dem Oberbegriff des Hauptanspruches.

Zur Beseitigung von im Körper eines Lebewesens befindlichen Konkrementen, beispielsweise Nieren- oder Gallensteinen, werden sogenannte Lithotriptoren eingesetzt, mit denen diese Konkremente mittels fokussierter Ultraschall-Stoßwellen berührungslos zertrümmert werden. Durch den Einsatz von Ultraschall-Stoßwellen können operative Eingriffe oder das Einführen von Sonden in den Körper des Patienten und die damit verbundene Infektionsgefährdung vermieden werden.

Um zu verhindern, daß gesundes Gewebe in der Umgebung des Konkrements beschädigt wird, muß das zu zerstörende Konkrement möglichst exakt im Fokus der Stoßwelle positioniert werden. Die zur exakten Positionierung erforderliche räumliche Ortung des Konkrementes kann dabei beispielsweise mittels Röntgenstrahlen oder Ultraschall erfolgen.

Ein wesentlicher Vorteil einer Ultraschall-Ortung des zu zertrümmernden Konkrementes ist darin zu sehen, daß zugleich mit der Ortung Information über die Bedingungen für die Schallausbreitung der Stoßwelle gewonnen werden kann, wenn das von der Ultraschall-Stoßwelle beschallte Gebiet im Körper des Lebewesens auch von den zur Ortung verwendeten Ultraschallwellen erfaßt wird.

Aus der DE-PS 27 22 252 sind beispielsweise Vorrichtungen zur räumlichen Ultraschall-Ortung von Konkrementen bekannt, bei denen die Laufzeit der von einem Ultraschall-Sender ausgesendeten und am Konkrement reflektierten oder gestreuten Impulse zur Lokalisierung des Konkrementes herangezogen wird. In einer Ausführungsform sind in der Wand eines Koppelgerätes, das die Stoßwellenquelle enthält und auf die Körperoberfläche eines Patienten aufgesetzt werden kann, ein Ultraschall-Sender und ein Ultraschall-Empfänger angeordnet, deren Rotationsachsen sich im Fokus der Ultraschall-Stoßwelle unter einem Winkel von 30° schneiden. Anstelle der Verwendung eines separaten Ultraschall-Senders ist auch eine Anordnung vorgesehen, bei der die mit verminderter Intensität betriebene Stoßwellenquelle selbst als Ultraschall-Sender zur Ortung des Konkrementes verwendet wird. Mittels in der Wand des Koppelgerätes angeordneten Drucksensoren werden dann die vom Konkrement ausgehenden gestreuten Impulse empfangen und aus den Laufzeitdifferenzen die Lage des Konkrementes ermittelt. In einer weiteren Ausführungsform ist ein schwenkbarer Ultraschallwandler vorgesehen, der in einer den Fokus der Ultraschall-Stoßwelle enthaltenden Ebene ein Schnittbild nach dem B-Bild-Verfahren erzeugt.

Im Hinblick auf eine sichere und einfache Positionierung des Konkrementes im Fokus der Ultraschall-Stoßwelle ist dabei von den bekannten Vorrichtungen zur Ultraschall-Ortung die nach dem B-Bild-Verfahren arbeitende Vorrichtung gegenüber den nach dem A-Bild-Verfahren arbeitenden Einrichtungen den Vorteil, daß das zweidimensionale B-Bild einerseits einen anschaulichen Eindruck über die geometrischen Verhältnisse in der Umgebung des Konkrementes vermittelt und andererseits sicherer zu interpretieren ist als ein eindimensionales A-Bild.

Echographische B-Bild-Verfahren haben jedoch den Nachteil, daß nur Schnittebenen eines Körpers dargestellt werden können, die senkrecht zur Körperachse und somit im wesentlichen parallel zur Ausbreitungsrichtung der Ultraschall-Schallwellen verlau fen. Dem Benutzer liegt somit für die Positionierung des Fokus oder des Konkrementes eine Bildinformation aus einer zur Schärfeneinstellung ungewohnten und wenig geeigneten Objektebene vor. So entspricht beispielsweise bei der bekannten Vorrichtung ein im Bild gegenüber dem Fokus lateral versetztes Konkrement einer Defokussierung, die bezogen auf die Ausbreitungsrichtung der Ultraschall-Stoßwelle sowohl aus einem lateralen als auch aus einem axialen Versatz bestehen kann.

Ein weiterer Nachteil der bekannten Vorrichtung ist auch darin zu sehen, daß insbesondere das durch die Breite der zur Abtastung verwendeten Schallkeule bedingte laterale Auflösungsvermögen eines echographischen Schnittbildes unbefriedigend ist und eine genaue axiale Ortung und Positionierung des Konkrementes im Fokus der Ultraschall-Stoßwelle erschwert.

Aus "Acoustical Holography Vol. 5, Plenum Publ.Corp., New York 1973, Ed. P.S.Green, Seiten 493 bis 503 ist eine Ultraschall-Abbildungsvorrichtung für diagnostische Zwecke bekannt, die nach dem Prinzip eines optischen Bildwerfers arbeitet. Diese als Ultraschall-Kamera bezeichnete Vorrichtung ermöglicht die Darstellung von Körperschnittbildern, die senkrecht zu den sagittalen Schnittebenen der Ultraschall-Echographie verlaufen. Dazu wird der zu untersuchende Körper mit Ultraschall "beleuchtet" und entweder die vom Körper gestreuten transmittierten oder die vom Körper gestreuten reflektierten Ultraschallwellen mittels eines Linsensystems auf einer Bildebene abgebildet und von einem dort angeordneten linearen Empfangs-Array in elektrische Signale umgewandelt. Mit Hilfe zweier im Strahlengang angeordneter und gegenläufig rotierender Prismen wird das vom Linsensystem erzeugte Bild sinusförmig abgelenkt, so daß mit den am linearen Empfangs-Array gemessenen Empfangssignalen ein zweidimensionales Bild aufgebaut werden kann. Die auf diese Weise erreichte Bildfrequenz beträgt etwa 15 Hz, so daß bereits von einer Echtzeit-Bilddarstellung gesprochen werden kann. Eine höhere Bildfrequenz ist jedoch nicht mehr möglich, da die hierzu erforderliche höhere Rotationsfrequenz der Prismen zu Turbulenzen innerhalb der schalltragenden Flüssigkeit und somit zu einer Störung der Schallausbreitung und zu einer Verminderung der Bildqualität führen würde. Die Tiefenlage der von der Ultraschall-Kamera abgebildeten Schnittebene ergibt sich entsprechend den Gesetzen der geometrischen Optik aus den Abbildungseigenschaften der verwendeten akustischen Abbildungsvorrichtungen, der Bildweite des Empfangs-Arrays und der Lage des Körpers relativ zu dem dann festgelegten Schärfenbereich im Gegenstandsraum.

Eine Ultraschall-Transmissionskamera, mit der eine höhere Bildfrequenz ohne mechanisch bewegte Teile bei vereinfachtem Aufbau der Abbildungsvorrichtung ermoglicht wird, ist beispielsweise in "Acoustical Imaging, Vol. 15, Plenum Publ. Corp., New York 1987, Ed. H.W.Jones, Seiten 213 bis 225" offenbart. Dort ist als Ultraschall-Empfänger anstelle eines linearen Empfangs-Arrays eine zweidimensionale Empfangs-matrix aus 29 x 128 Wandlerelementen vorgesehen. Die an den einzelnen Wandlerelementen anstehenden elektrischen Signale werden nacheinander gelesen und zu einem zweidimensionalen Bild zusammengefügt, dessen Bildfrequenz etwa 25 Hz beträgt. Der ultraschallempfindliche Bereich der Empfangsmatrix wird dabei aus einer dünnen PVDF-Folie gebildet, die gegen eine matrixförmige Elektrodenanordnung gepreßt wird. Eine Weiterentwicklung einer derartigen Empfangsmatrix ist beispielsweise auch aus der US-PS 4 742 494 bekannt.

Der Erfindung liegt nun die Aufgabe zugrunde, eine Vorrichtung zur Ultraschall-Ortung von im Körper eines Lebewesens befindlichen Konkrementen anzugeben, die eine einfache und genaue Positionierung des Konkrementes im Fokus einer Ultraschall- Stoßwelle ermöglicht.

Die genannte Aufgabe wird erfindungsgemäß gelöst mit den kennzeichnenden Merkmalen des Hauptanspruchs.

Mit einer Vorrichtung zur Ultraschall-Ortung, die nach dem Prinzip einer in Reflexion betriebenen Ultraschall-Kamera arbeitet, wird dem Benutzer eine Ortungsvorrichtung zur Verfügung gestellt, die während der Positionierung des Konkrementes im Fokus der Ultraschall-Stoßwelle ein Echtzeit-Bild einer außerhalb der Sagittalebene liegenden Schnittebene des Körpers erzeugt, mit dessen Hilfe die genaue Positionierung erleichtert wird. Insbesondere kann eine Schnittebene des Körpers ausgewählt werden, die parallel zur Fokalebene der Ultraschall-Stoßwelle verläuft. Dem Benutzer liegt damit eine Bildinformation aus einer zur Schärfeneinstellung des Lithotriptors besonders geeigneten Objektebene vor. Die Abbildungsbedingungen der Ultraschall-Kamera sind dabei so eingestellt, daß ein Gebiet des Gegenstandsraumes scharf abgebildet wird, das den Fokus der Ultraschall-Stoßwelle enthält, so daß der Fokus einem vorbestimmten Bildbereich auf einem Monitor, beispielsweise der Bildmitte, fest zugeordnet ist. Dem Betrachter erscheint dabei das zu zertrümmernde Konkrement nur dann scharf und in der Bildmitte, wenn es auch tatsächlich im Fokus der Ultraschall-Stoßwelle liegt. Jede tatsächliche axiale Dejustierung außerhalb des Bereiches der Tiefenschärfe ist als Unschärfe auf dem Monitorbild erkennbar. Bedingt durch das bei der Ultraschall-Kamera verwendete Abbildungsprinzip ist das Auflösungsvermögen in jeder lateralen Richtung innerhalb der Schnittebene gleich groß. Da außerdem mit einer Ultraschall-Kamera ein für den Betrachter plastisches Echtzeit-Bild mit einer hohen lateralen Auflösung erzeugt werden kann, ist die genaue Positionierung des Konkrementes im Fokus der Ultraschall-Stoßwelle erheblich erleichtert.

Weitere vorteilhafte Ausgestaltungen der Erfindung ergeben sich gemäß der Unteransprüche.

Zur weiteren Erläuterung der Erfindung wird auf die Zeichnung verwiesen, in deren

Figur 1 die erfindungsgemäße Vorrichtung zur Ultraschall-Ortung veranschaulicht ist und in deren

Figuren 2, 3 und 4 weitere vorteilhafte Ausgestaltungen der Erfindung schematisch dargestellt sind.

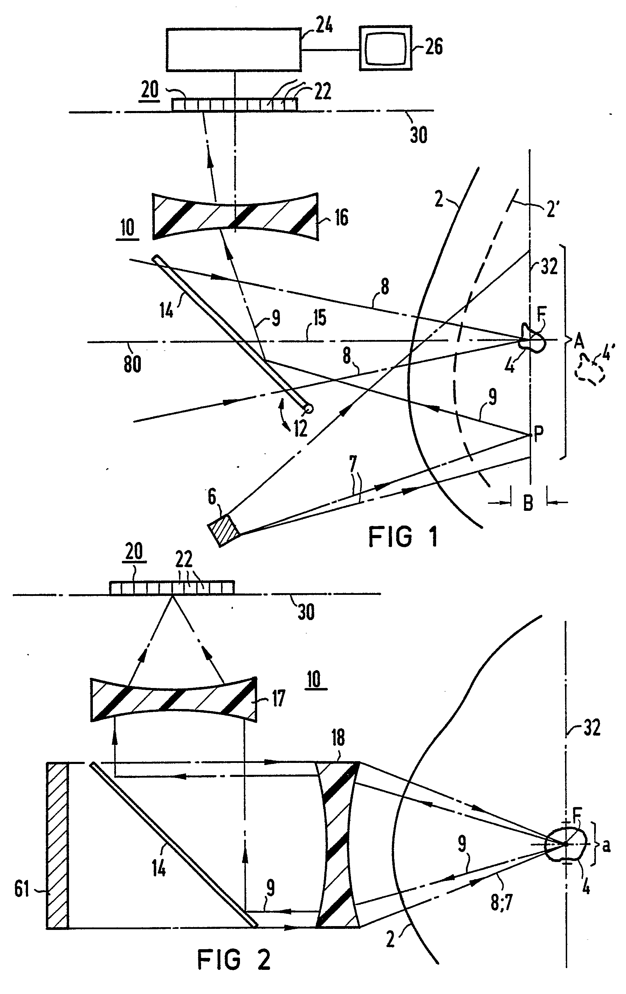

Gemäß Figur 1 befindet sich im Körper 2 eines Lebewesens ein Konkrement 4, das mit Hilfe einer Ultraschall-Stoßwelle 8 zertrümmert werden soll. Diese Ultraschall-Stoßwelle 8 wird von einer in der Figur nicht dargestellten fokussierten Stoßwellen-Sender eines Lithotriptors erzeugt. Die Ortung des Konkrementes 4 erfolgt mit einer in Reflexion betriebenen Ultraschall-Kamera, die einen Ultraschall-Sender 6 zur kontinuierlichen oder gepulsten Beschallung eines den Fokus F der Ultraschall-Stoßwelle 8 enthaltenden Raumgebietes des Körpers 2 mit Ultraschallwellen 7, eine Abbildungsvorrichtung 10 sowie einen Ultraschall-Empfänger 20 enthält, der in einer Bildebene 30 der Abbildungsvorrichtung 10 angeordnet ist. Der Ultraschall-Empfänger 20 enthält eine Vielzahl von Wandlerelementen 22 und ist zur Signalauswertung mit einer Steuer- und Auswerteeinrichtung 24 verbunden, die die am Ultraschall-Empfänger 20 empfangene Schalldruck-Verteilung als Bild auf dem Bildschirm eines Monitors 26 ausgibt. Der Ultraschall-Empfänger 20 enthält in einer vorteilhaften Ausgestaltung der Erfindung eine Vielzahl von Wandlerelementen 22, die in Gestalt einer Matrix angeordnet sind. Eine besonders geeignete Wandleranordnung ist beispielsweise aus der US-PS 4 742 494 bekannt. Stoßwellen-Sender und Ultraschall-Kamera befinden sich beispielsweise in einem in der Figur nicht dargestellten Koppelgerät, das mit einer schalltragenden Flüssigkeit gefüllt ist und über einen elastischen Gummibalg auf die Oberfläche des Körpers 2 aufgesetzt wird. In einer vorteilhaften Ausgestaltung der Erfindung ist in der Wand des Koppelgerätes am Ort des Ultraschall-Empfängers 20 eine mit einer PVDF-Folie verschlossene Öffnung vorgesehen, an die von außen eine Elektrodenmatrix in Analogie zu der aus der DE-OS 36 28 705 bekannten Vorrichtung angedrückt wird.

Die Abbildungsvorrichtung 10 besteht im Beispiel der Figur aus einem akustischen Strahlteiler 14, beispielsweise einer planparallelen dünnen Platte aus Polystyrol PS, und einer akustischen Linse 16, vorzugsweise ebenfalls aus Polystyrol PS, die die vom Körper 2 reflektierten Ultraschallwellen 9 auf die Empfangsfläche des Ultraschall-Empfängers 20 abbildet. Der Strahlteiler 14 ist vorzugsweise unter einem Winkel von 45° gegen die Mittelachse 80 der Ultraschall-Stoßwelle 8 im Strahlengang der Ultraschall-Stoßwelle 8 angeordnet, so daß der Strahlengang innerhalb der Ultraschall-Kamera mit dem Strahlengang der Ultraschall-Stoßwelle 8 wenigstens im Körper 2 verschränkt ist. Durch diese Verschränkung können bei der Ultraschall-Ortung auch Strukturen erfaßt werden, die zwischen der Oberfläche des Körpers 2 und dem Fokus F der Ultraschall-Stoßwelle 8 liegen und einen störenden Einfluß auf die Ausbreitung der Ultraschall-Stoßwellen 8 ausüben könnten. Der Strahlteiler 14 ist dabei wenigstens während der Ortung im Strahlengang der Ultraschall-Stoßwelle 8 angeordnet. Während des Beschießens des Konkrementes 4 mit der Ultraschall-Stoßwelle 8 ist er in einer bevorzugten Ausführungsform aus dem Strahlengang entfernt. Gemäß dem Beispiel der Figur ist hierzu vorzugsweise ein um eine Drehachse 12 schwenkbarer Strahlteiler 14 vorgesehen. Bei einem entsprechend der Figur versetzt zum Strahlengang der Ultraschall-Stoßwelle 8 angeordneten Ultraschall-Sender 6 kann zur Ortung anstelle eines Strahlteilers 14 auch ein akustisch undurchlässiger Spiegel, beispielsweise eine Metallplatte, in den Strahlengang der Ultraschall-Stoßwelle 8 eingeschwenkt oder eingeschoben werden.

Die Brennweite der akustischen Linse 16 und die Bildweite des Ultraschall-Empfängers 20 sind so gewählt, daß der Schärfenbereich B der Ultraschall-Kamera auf eine Gegenstandsebene 32 eingestellt ist, die den Fokus F der Ultraschall-Stoßwelle 8 enthält. Bedingt durch das endliche laterale Auflösungsvermögen des Ultraschall-Empfängers 20, das in allen lateralen Richtungen etwa 2 mm beträgt, und die, bezogen auf die geometrischen Abmessungen der Abbildungsvorrichtung 10, bereits nicht mehr vernachlässigbare Schallwellenlänge, wird nicht nur eine Gegenstandsebene 32 scharf abgebildet, die sich in Analogie zu den Gesetzen der geometrischen Optik ergeben würde, sondern ein Schärfenbereich B, dessen Tiefe je nach den Wellenlängen der vom Ultraschall-Sender 6 gesendeten Ultraschallwellen 7 etwa 10 mm bis 20 mm beträgt. Die innerhalb dieses Schärfenbereiches B liegende Umgebung eines jeden Punktes P, der sich auf der Gegenstandsebene 32 befindet und von dem eine gestreute oder reflektierte Ultraschallwelle 9 ausgeht, wird somit am Ultraschall-Empfänger 20 scharf abgebildet.

In der bevorzugten Ausführungsform gemäß der Figur fällt die Mittelachse 80 der Ultraschall-Stoßwelle 8 mit der im Gegenstandsraum zwischen dem Körper und dem Strahlteiler verlaufenden Abbildungsachse 15 der Abbildungsvorrichtung 10 zusammen, so daß die Gegenstandsebene 32 senkrecht auf der Mittelachse 80 steht. Das am Monitor 26 dargestellte Schnittbild des Körpers 2 entspricht dann einer Schnittebene des Körpers die senkrecht zur Ausbreitungsrichtung der Ultraschall-Stoßwelle 8 verläuft, so daß jeder tatsächlichen lateralen Dejustierung des Konkrementes 4 in der Fokalebene eine durch den Abbildungsmaßstab der Abbildungsvorrichtung 10 gegebene laterale Verschiebung des weiterhin scharf abgebildeten Konkrementes auf dem Bildschirm des Monitors 26 entspricht. Das Positionieren eines außerhalb des Fokus F befindlichen Konkrementes 4' kann dann in der aus der Optik bei Schärfeneinstellung gewohnten Weise erfolgen, in dem beispielsweise zunächst durch eine axiale Relativverschiebung des Körpers 2' und der mit dem Lithotriptor starr verbundenen Ultraschall-Kamera auf dem Monitor 26 ein scharfes Bild des Konkrementes 4' erzeugt wird, das dann durch laterale Verschiebungen, die direkt als ebenfalls laterale Verschiebungen auf dem Monitor angezeigt werden, in die beispielsweise der markierten Bildmitte des Monitors 26 entsprechenden Fokuszone der Ultraschall-Stoßwelle 8 geführt wird. Außerdem ist durch die Anordnung gemäß der Figur gewährleistet, daß die von der Gegenstandsebene 32 ausgehende Ultraschallwelle 9 das gleiche Raumgebiet im Körper 2 durchquert wie die Ultraschall-Stoßwelle 8.

Gemäß Figur 2 ist eine Ausführungsform vorgesehen, bei der der zur Ultraschall-Ortung verwendete Ultraschall-Sender und der zur Erzeugung der Ultraschall-Stoßwelle 8 verwendete Stoßwellen-Sender durch einen gemeinsamen, beispielsweise piezoelektrischen Ultraschall-Sender 61 gebildet wird, der zur Ortung Ultraschallwellen 7 im cw-Betrieb erzeugt, deren Intensität gegenüber der Intensität der Ultraschall-Stoßwelle 8 verringert ist. In dieser Anordnung wird im Gegensatz zur Anordnung nach Figur 1 auch bei der Ortung nur ein kleines Gebiet der der Bildebene 30 zugeordneten Gegenstandsebene 32 ausgeleuchtet, dessen laterale Ausdehnung a jedoch auch in Fokusnähe ausreicht um das Konkrement 4 scharf in der Bildmitte des Ultraschall-Empfängers 20 abzubilden. Der Umstand, daß die Lage des Fokus der Ultraschallwelle 7 gegenüber der Lage des Fokus der Ultraschall-Stoßwelle 8 axial verschoben sein kann, spielt dabei keine Rolle, da der eingestellte Schärfenbereich der Ultraschall-Kamera unabhängig vom Fokus der Ultraschallwelle 7 ist.

Bei einem ebenen Ultraschall-Sender 61 ist der akustische Strahlteiler 14 während der Ultraschall-Ortung vorzugsweise zwischen dem Ultraschall-Sender 61 und einer zur Fokussierung der Ultraschall-Stoßwelle 8 dienenden Linse 18 angeordnet, so daß diese gemeinsam mit einer Linse 17 Teil der Abbildungsvorrichtung 10 der Ultraschall-Kamera ist. Diese Anordnung hat den Vorteil, daß sie zugleich eine Überwachung der Lage des Fokus F der Ultraschall-Stoßwelle 8 ermöglicht. Bei Betrieb des Lithotriptors kann es nämlich beispielsweise vorkommen, daß durch die von den Ultraschall-Stoßwellen hervorgerufenen mechanischen Belastungen eine Verschiebung des Ultraschall-Senders 61 oder der Linse 18 in ihren Halterungen hervorgerufen wird. Eine solche Dejustierung kann zur Folge haben, daß sich der Brennfleck der Ultraschall-Stoßwelle 8 außerhalb des ursprünglichen Fokus befindet. Da jedoch der Ultraschall-Sender 61 und die Linse 18 Teile der Ultraschall-Kamera sind, führt dies dazu, daß bei der Ultraschall-Ortung das beleuchtete Raumgebiet auf die Empfangsfläche des Ultraschall-Empfängers 20 nicht mehr symmetrisch um dessen Mitte abgebildet wird. Der Betrachter kann somit bereits bei der Ultraschall-Ortung eine Dejustierung des Fokus des Lithotriptors feststellen und gegebenenfalls Maßnahmen zur Korrektur der Fokuslage ergreifen.

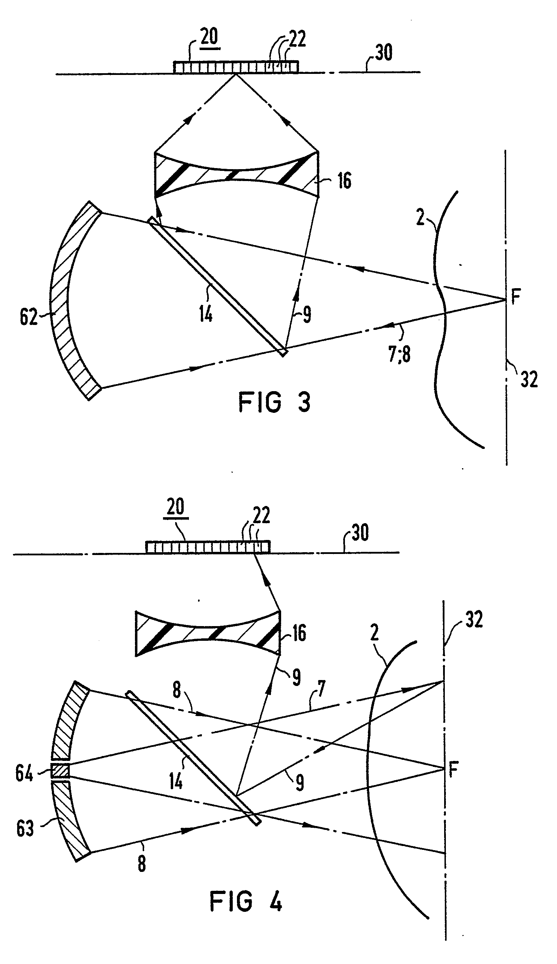

Dies ist auch bei einer Anordnung gemäß Figur 3 möglich, bei der die fokussierte Ultraschall-Stoßwelle 8 mittels eines kalottenförmigen Ultraschall-Senders 62 erzeugt wird, so daß eine Linse zur Fokussierung der Ultraschall-Stoßwelle 8 nicht mehr erforderlich ist.

Bei der Anordnung gemäß Figur 4 ist für die Ultraschall-Kamera ein Ultraschall-Sender 64 vorgesehen, der in einer zentralen Bereich eines ihn umgebenden Stoßwellen-Senders 63 angeordnet ist. Im Beispiel der Figur ist der Ultraschall-Sender 64 in einer zentralen Bohrung eines kalottenförmigen Stoßwellen-Senders 63 eingesetzt. Diese Anordnung hat den Vorteil, daß wie bei der Ausführungsform gemäß Figur 1 zur Aufnahme des Ultraschall-Bildes ein großes Gebiet des Gegenstandsraumes ausgeleuchtet wird und durch die damit verbundene größere auf dem Monitor abgebildete Schnittebene des Körpers 2 die Suche nach dem Konkrement erleichtert wird. Durch diese Anordnung wird außerdem eine nahezu vollständige Überlappung des von der Ultraschall-Welle 7 beschallten Gebietes mit den von der Ultraschall-Stoßwelle 8 beschallten Gebiet erreicht und Strukturen, die die Ausbreitung der Ultraschall-Stoßwelle 8 stören könnten, werden sicherer erfaßt.

Als gemeinsame Ultraschall-Sender für den Lithotriptor und die Ultraschall-Kamera eignen sich auch ring- oder matrixförmige Wandlerarrays, wie sie beispielsweise in der US-PS 4 526 168 offenbart sind und bei denen die einzelnen Wandlerelemente der Wandlerarrays bzw. Fokussierung der Ultraschall-Stoßwelle getrennt voneinander und phasenverzögert angesteuert werden. Für die Ultraschall-Ortung ist es dann ausreichend, wenn nur wenige der Wandlerelemente, beispielsweise nur ein einzelnes zentrales Wandlerelement, zur Beschallung des Körpers 2 verwendet werden.

**Ansprüche**

1. Vorrichtung zur räumlichen Ultraschall-Ortung von im Körper (2) eines Lebewesens befindlichen Konkrementen (4), die zur Zertrümmerung in einem Lithotriptor im Fokus (F) einer Ultraschall-Stoßwelle (8) angeordnet werden, **dadurch gekennzeichnet,** daß zur Ultraschall-Ortung eine in Reflexion betriebene Ultraschall-Kamera vorgesehen ist, deren Schärfenbereich (B) auf eine Gegenstandsebene (32) eingestellt ist, die wenigstens annähernd den Fokus (F) der Ultraschall-Stoßwelle (8) enthält.

2. Vorrichtung nach Anspruch 1, **dadurch gekennzeichnet,** daß die Ultraschall-Kamera einen Ultraschall-Empfänger (20) enthält, der aus einer Vielzahl von in Gestalt einer Matrix angeordneten Wandlerelementen (22) besteht.

3. Vorrichtung nach einem der Ansprüche 1 oder 2, **dadurch gekennzeichnet,** daß die Mittelachse (80) der Ultraschall-Stoßwelle (8) mit der im Gegenstandsraum befindlichen Abbildungsachse (15) der Ultraschall-Kamera wenigstens annähernd zusammenfällt.

4. Vorrichtung nach Anspruch 3, **dadurch gekennzeichnet,** daß im Strahlengang der Ultraschall-Stoßwelle (8) ein akustischer Strahlteiler (14) angeordnet ist.

5. Vorrichtung nach einem der Ansprüche 4 oder 5, **dadurch gekennzeichnet,** daß ein schwenkbarer Strahlteiler (14) vorgesehen ist.

6. Vorrichtung nach Anspruch 4, **dadurch gekennzeichnet,** daß zum Erzeugen der Ultraschallwellen (7) für die Ultraschall-Ortung und zum Erzeugen der Ultraschall-Stoßwelle (8) ein gemeinsamer Ultraschall-Sender (61) vorgesehen ist.

7. Vorrichtung nach Anspruch 6, **dadurch gekennzeichnet,** daß der akustische Strahlteiler (14) zwischen einem ebenen Ultraschall-Sender (61) und einer Linse (18) zur Fokussierung der Ultraschall-Stoßwelle (8) angeordnet ist.

8. Vorrichtung nach Anspruch 4, **dadurch gekennzeichnet,** daß zur Ultraschall-Ortung ein Ultraschall-Sender (64) vorgesehen ist, der im zentralen Bereich eines ihn umgebenden Stoßwellen-Senders (63) angeordnet ist.

9. Vorrichtung nach Anspruch 5, **dadurch gekennzeichnet,** daß ein gemeinsamer Ultraschall-Sender mit mehreren Wandlerelementen vorgesehen ist, die getrennt voneinander ansteuerbar sind.

88 P 3215

FIG 1

FIG 2

88 P 3215

FIG 3

FIG 4